# EUROPEAN PATENT SPECIFICATION

(11) **EP 0 808 111 B1**
(45) Date of publication and mention of the grant of the patent: **01.09.1999**
(21) Application number: 96903676.3
(22) Date of filing: 29.01.1996
(51) Int. Cl.: A23K 1/16, A61K 31/59

(54) **A LOW PHOSPHORUS ANIMAL FEED CONTAINING 1 -ALPHA- HYDROXYLATED VITAMIN D COMPOUNDS**
1-ALPHA-HYDROXY-VITAMIN D - VERBINDUNGEN ENTHALTENDES TIERFUTTER MIT NIEDRIGEM PHOSPHORGEHALT
ALIMENT POUR ANIMAUX A FAIBLE TENEUR EN PHOSPHORE CONTENANT DES COMPOSES DE LA VITAMINE D 1-ALPHA-HYDROXYLES

(30) Priority: 06.02.1995 US 383952
(43) Date of publication of application: 26.11.1997
(73) Proprietor: WISCONSIN ALUMNI RESEARCH FOUNDATION, Madison, WI 53705 (US)
(72) Inventor: DELUCA, Hector, F., Deerfield, WI 53531 (US); BAKER, David, H., Champaign, IL 61821 (US)
(74) Representative: Ellis-Jones, Patrick George Armine
(86) International application number: US9601021
(87) International publication number: WO9624258

(56) References cited:
- EP-A- 0 383 116
- WO-A-93/19759
- ZEITSCHRIFT FÜR VERSUCHSTIERKUNDE, vol. 27, no. 3/4, 1985, pages 163-168, XP002001500 ERLING TVEDEGAARD: "Absorption of calcium, magnesium and phosphate during chronic renal failure and the effect of vitamin D in rabbits"
- JOURNAL OF NUTRITION, vol. 125, no. 9, 1995, pages 2407-2419, XP002001501 ROBERT R. BIEHL ET AL.: "1-alpha-hydroxylated cholecalciferol compounds act additively with microbial phytase to improve phosphorus, zinc and manganese utilization in chicks fed soy-based diets" cited in the application
- POULTRY SCIENCE, vol. 73, no. 8, 1994, pages 1312-1326, XP002001502 KEVIN D. ROBERSON ET AL.: "Effects of 1,25-dihydoxycholecalciferol and phytase on zinc utilization in broiler chicks"
- POULTRY SCIENCE, vol. 69, no. 3, 1990, pages 426-432, XP002001503 R.H. HARMS ET AL.: "Some observations on the influence of vitamin D metabolites when added to the diet of commercial laying hens"
- JOURNAL OF DAIRY SCIENCE, vol. 65, no. 10, 1982, CHAPAIGN, ILLINOIS US, pages 1934-1940, XP002001504 K. HOVE ET AL.: "Prevention of parturient hypocalcemia : effect of a single oral dose of 1,25-dihydroxyvitamin D3"
- POULTRY SCIENCE, vol. 74, no. 1, 1995, pages 121-126, XP002001505 SEIJI AOYAGI ET AL.: "Effect of microbial phytase and 1,25-dihydroxycholecalciferol on dietary copper utilization in chicks"

## Description

### Background and Summary of the Invention

Up to 80% of the phosphorus (P) present in plant foods and feeds exists as a complex of phytic acid (myoinositol hexaphosphate), hereinafter referred to as phytate. Phytate may structurally be illustrated by the following formula: 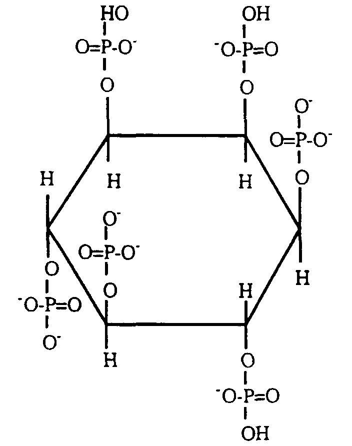 The P in phytate cannot be totally digested by simple-stomached animals, including humans, and it therefore passes through the gastrointestinal (GI) tract and is excreted in the feces. In animal nutrition, this is accounted for in diet formulation whereby 1.5 to 2.0% of an inorganic phosphate source is supplemented to meet the animal's minimal P requirement. Addition of inorganic P to poultry, swine, companion animal, and fish diets is expensive. It is often stated that supplemental P for these species is the third most expensive dietary ingredient, after energy and protein. The body requires P for formation of bones and teeth, for phospholipid (cell membrane structure) and nucleic acid (RNA, DNA) synthesis, for synthesis of ATP and other high-energy P compounds, and for proper acid-base balance in the body. Roughly 85% of the body P is in the skeleton. Bone is comprised of 50% organic matrix (protein in the form of collagen, and lipid) and 50% inorganic material (mostly a Ca-P salt. i.e., hydroxyapatite).

Supplemental inorganic P is provided to animal diets in one of three feedgrade forms; dicalcium phosphate (18.5% P), monocalcium phosphate (21.5% P) or deflorinated phosphate (18.0% P). The combined total market for these products is estimated to be 675 million dollar's per year in the U.S., Canada, Mexico, Western Europe and Japan. If one were to include South America, Eastern Europe, Asia, Africa, China, India, and Southeast Asia, (where market data are difficult to obtain), the total market for feed-grade phosphates could easily be expected to exceed 1 billion dollars annually. In North America 50% of feed-grade phosphate consumed is used for poultry feeding. It has been discovered that use of a bioactive 1-α-OH vitamin D compound would reduce the need for supplemental P by up to 40%, and if combined with the enzyme phytase, could reduce the need by up to 50% to 60%.

Phytate complexes in plant foods and feeds (eg.. cereal grains and by-products, beans) also bind cations such as calcium, potassium, magnesium, zinc, iron and manganese (Erdman, 1979) illustrated schematically as follows: 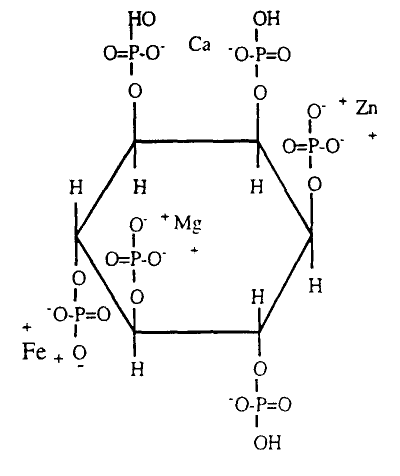 A bioactive feed additive that causes the utilization of P from phytate should also increase utilization of these elements as well. The present invention has established that 1-α-OH vitamin D compounds, preferably 1,25 dihydroxycholecalciferol and 1-α-OH cholecalciferol, increase the utilization of not only P but also zinc, iron and manganese. Thus, because these three trace elements are always added in supplemental form to diets for swine, poultry and companion animals (as feed-grade ZnO or ZnSO₄·H₂O; FeSO₄·H₂O; MnO or MnSO₄·H₂O) use of a bioactive 1-α-OH vitamin D compound would lower, or perhaps eliminate, the need for supplemental quantities of these mineral salts in a practical-type grain-oilseed meal diet.

By replacing up to 0.75% of the diet as a P supplement and up to 0.10% as trace mineral salts, the remaining diet would contain more usable energy. Thus, grain-oilseed meal diets generally contain about 3,200 kcal metabolizable energy per kilogram of diet, and mineral salts supply no metabolizable energy. Removal of the unneeded minerals and substitution with grain would therefore increase the usable energy in the diet.

Currently, phytase is being used in much of Europe and Asia to reduce P pollution. The use level, however, is 600 units per kilogram diet, but this level was selected because of cost of the erzyme and not because 600 units will maximize phytate utilization. In contrast it has been discovered via the present investigation that at least 1200 units/kg diet is required to maximize phytate utilization in chicks fed a corn-soybean meal diet (Table 1). However, use of a bioactive 1-α-OH vitamin D compound in accordance with the present invention would reduce the need to feed expensive levels of phytase. (Table 5)

Animal producers are forced to feed high P diets because of the phytate content of diets. This increases P in the excreta waste products (both feces and urine). Excess P from animal, as well as human waste, is generally spread on the soil, where a portion of it gets washed into ground water and then into ponds, streams, rivers, lakes and oceans. Too much P in water stimulates growth of algae, and algae take up considerable oxygen. This robs marine life of the oxygen they need to grow, reproduce and thrive.

In many parts of Europe and Asia, P pollution has become such a problem and concern that penalties in the form of stiff financial fines are imposed on livestock producers who spread too much P-laden manure on the soils. Because of this, much of Europe now uses a microbial phytase product (BASF), even though this product (which also hydrolyses phytate) is very expensive, in fact too expensive to be cost effective (at 600 units/kg diet) as a feed additive in the U.S. at the present time. Many U.S. soils are being described as "P saturated", thus resulting in a greater concentration of P in soil leachates. High-P water leachate in areas such as the Chesapeak Bay has been blamed for excessive algae growth and increased fish kills in bay waters (Ward, 1993). In Europe, the feed industry group FEFANA issued a position paper in 1991 entitled "Improvement of the Environment". They proposed that P in manure from livestock production should be reduced by 30% (Ward, 1993). The limits of P that can be applied to soils in Europe have been discussed by Schwarz (1994). Accordingly, it is estimated that use of a 1-α-OH vitamin D compound that is active in increasing phosphorus utilization in accordance with the present invention, could cut the P content of animal waste products by up to 40%.

Initial work focused on use of 1,25 dihydroxycholecalciferol (1,25-(OH)₂D₃) in the absence or presence of 1200 units of microbial phytase (BASF), WO 93/19759 discloses that 1,25-(OH)₂D₃ is effective in improving P utilization from phytatebound P, and Biehl et al (1995) confirmed his results. Moreover, both studies showed that 1,25-(OH)₂D₃ works additively with microbial phytase in releasing P from dietary phytate complexes. It seems likely that 1,25-(OH)₂D₃ exerts is effects in two ways: (a) the 1,25 compound likely increases the activity of intestinal phytases or phosphatases that hydrolyze phytate (Pileggi et al, 1955; Maddaiah et al, 1964) and (b) the 1,25 compound is known to stimulate phosphate transport (Tanka and DeLuca, 1974), facilitating transport of P from GI tract to plasma and hence bone. Zeitschrift Für Versuchstierkunde, 27, No. 3/4 , 1985, pages 163-168 discloses a feed composition for rabbits comprising 1α-OH D₃, 0.9% phosphorus and other ingredients.

Under normal dietary circumstances, cholecalciferol (vitamin D₃) that is added to a diet gets absorbed from the GI tract and is transported via blood to the liver where the liver enzyme 25-hydroxylase acts on the compound to cause formation of 25-OH D₃. This compound is the normal blood metabolite of cholecalciferol. A small portion of 25-OH D₃ undergoes a further hydroxylation step in the kidney, at the 1-α position, causing synthesis of the calciotropic hormone 1,25-(OH)₂D₃. Because 1,25-(OH)₂D₃ is expensive to synthesize and because oral 25-OH D₃ is not the active form in phosphate absorption, it was proposed that 1-α-OH D₃ would be an effective compound for increasing phosphate utilization. It has been discovered that 1α-hydroxylated vitamin D compounds and particularly 1-α-OH D₃ will be absorbed from the GI tract and then be transported to the liver where 25-hydroxylase would act upon it to bring about synthesis of 1,25-dihydroxylated compounds and particularly 1,25-(OH)₂D₃. A portion of these compounds would then be transported back to the GI tract where they would activate intestinal phosphate absorption. The net effect would be an increased utilization of P (also Zn, Fe, Mn and Ca) from the phytate complex.

In summary, the potential benefits of the present invention include (1) reduction in the need for inorganic P supplements for animal (including fish) diets; (2) reduction in P pollution of the environment; (3) reduction or possible elimination of the need for supplemental Zn, Mn and Fe in animal diets; and (4) reduction of the quantity of phytase needed for maximal P utilization from feeds.

### Detailed Description of the Preferred Embodiment

As used in the description, the term hydroxy-protecting group signifies any group commonly used for the temporary protection of hydroxy functions, such as for example, alkoxycarbonyl, acyl, alkylsilyl, and alkoxyalkyl groups, and a protected hydroxy group is a hydroxy function derivatized by such a protecting group. Alkoxycarbonyl protecting groups are groupings such as methoxycarbonyl, ethoxycarbonyl, propoxycarbonyl, isopropoxycarbonyl, butoxycarbonyl, isobutoxycarbonyl, tert-butoxycarbonyl, benzyloxycarbonyl or allyloxycarbonyl. The term "acyl" signifies an alkanoyl group of 1 to 6 carbons, in all of its isomeric forms, or a carboxyalkanoyl group of 1 to 6 carbons, such as an oxalyl, amlonyl, succinyl, glutaryl group, or an aromatic acyl group such as benzoyl, or a halo, nitro or alkyl substituted benzoyl group. The word "alkyl" as used in the description, denotes a straight-chain or branched alkyl radical of 1 to 10 carbons, in all its isomeric forms. Alkoxyalkyl protecting groups are groupings such as methoxymethyl ethoxyethyl, methoxyethoxymethyl, or tetrahydrofuranyl and tetrahydropyranyl. Preferred alkylsilyl protecting groups are trimethylsilyl, triethylsilyl, t-butyldimethylsilyl, and analogous alkylated silyl radicals.

The vitamin D compounds useful in the present treatment are 1α-hydroxylated vitamin D compounds, preferably 1a-hydroxycholecalciferol and 1α,25-dihydroxycholecalciferol. The vitamin D compounds of this type are characterized by the following general structure: 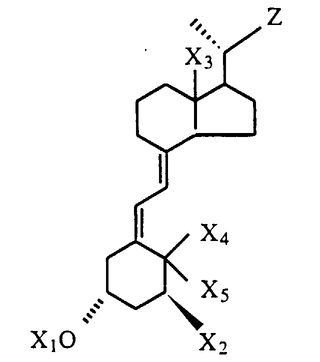 where X₁ may be hydrogen or a hydroxy-protecting group, X₂ may be hydroxy, or protected hydroxy, X₃ may be hydrogen or methyl, X₄ and X₅ each represent hydrogen or taken together X₄ and X₅ represent a methylene group, and where Z is selected from Y, -OY, -CH₂OY,-C≡-CY and-CH=CHY, where the double bond may have the cis or trans stereochemical configuration, and where Y is selected from hydrogen, methyl, -CR₅O and a radical of the structure: 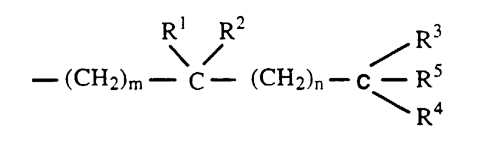 where m and n, independently, represent integers from 0 to 5, where R¹ is selected from hydrogen, hydroxy, protected-hydroxy, fluoro, trifluoromethyl, and C₁₋₅-alkyl, which may be straight chain or branched and, optionally, bear a hydroxy or protected-hydroxy substituent, and where each of R², R³ and R⁴, independently, is selected from hydrogen, fluoro, trifluoromethyl and C₁₋₅ alkyl, which may be straight-chain or branched, and optionally bear a hydroxy or protected-hydroxy substituent, and where R¹ and R², taken together, represent an oxo group or an alkylidene group, =CR₂R₃, or the group -(CH₂)ₚ-, where p is an integer from 2 to 5, and where R³ and R⁴, taken together, represent an oxo group, or the group -(CH₂)_{q}-, where q is an integer from 2 to 5, and where R⁵ presents hydrogen, hydroxy, protected-hydroxy, or C₁₋₅ alkyl.

The above compounds may be administered alone or in combination with other feed additive agents. The above vitamin D compounds or combinations thereof can be readily administered either by mixing them directly into animal feed or separately from the feed by separate oral dosage, by injection or by transdermal means or in combination with other 1α-hydroxylated vitamin D compounds, the proportions of each of the compounds in the combination being dependent upon the particular problem being addressed and the degree of response desired, are generally effective to practice the present invention. In poultry, amounts in excess of about 10 micrograms per day or the combination of that compound with other 1α-hydroxylated vitamin D compounds, are generally unnecessary to achieve the desired results, may result in hypercalcemia, and may not be an economically sound practice. It should be understood that the specific dosage administered in any given case will be adjusted in accordance with the specific compounds being administered, the problem to be treated, the condition of the subject and the other relevant facts that may modify the activity of the compound or the response of the subject, as is well known by those skilled in the art. In general, either a single daily dose or divided daily dosages may be employed, as is well known in the art.

If administered separately from the animal feed, dosage forms of the various compounds can be prepared by combining them with non-toxic pharmaceutically acceptable carriers to make either immediate release or slow release formulations, as is well known in the art. Such carriers may be either solid or liquid such as, for example, corn starch, lactose, sucrose, peanut oil, olive oil, sesame oil and propylene glycol. If a solid carrier is used the dosage form of the compounds may be tablets, capsules, powders, troches or lozenges or top dressing as microdispersable forms. If a liquid carrier is used, soft gelatin capsules or syrup or liquid suspensions, emulsions or solutions may be the dosage form. The dosage forms may also contain adjuvants, such as preserving, stabilizing, wetting or emulsifying agents, solution promoters, etc, They may also contain other therapeutically valuable substances.

The present invention also relates to an animal feed composition and method of compounding an animal feed utilizing a 1α-hydroxylated vitamin D compound to lower the dietary requirement of phosphorus in the animal feed. The 1α-hydroxylated vitamin D compounds suitable for this use have been previously described herein. The amount of a phosphorus supplement (18.5%P) that may be incorporated with the feed may be reduced to 0.9% or even 0.5% from 1.9% on a dry weight basis. This is a significant reduction from the normal amount of phosphorus supplement incorporated in animal feed compositions of 1.5% to 2.5%. This beneficial reduction in phosphorus is a direct result of the incorporation of a 1α-hydroxylated vitamin D compound in the animal feed.

The animal feed may be any protein-containing organic meal normally employed to meet the dietary requirements of animals. Many of such protein-containing meals are typically primarily composed of corn, soybean meal or a corn/soybean meal mix. For example, typical commercially available products fed to fowl include Egg Maker Complete, a poultry feed product of Land O' Lakes AG Services, as well as Country Game & Turkey Grower a product of Agwa, Inc. Both of these commercially available products are typical examples of animal feeds with which the present 1α-hydroxylated vitamin D compounds may be incorporated to reduce the amount of supplemental phosphorus. zinc, manganese and iron intake required in such compositions. Thus, any type of protein-containing organic meal may be utilized as the base mix to which the 1α-hydroxylated vitamin D compounds and reduced supplemental phosphorus, zinc, manganese and iron amounts of the present invention may be incorporated.

The present invention is applicable to the diet of numerous animals, which herein is defined as including mammals, fowl and fish. In particular, the diet may be employed with commercially significant mammals such as pigs, cattle, sheep, goats, laboratory rodents (rats, mice, hamsters and gerbils), fur-bearing animals such as mink and fox, and zoo animals such as monkeys and apes, as well as domestic mammals such as cats and dogs. Typical commercially significant fowl include chickens, turkeys, ducks, geese, pheasants and quail. Commercially farmed fish such as trout would also benefit from the diet disclosed herein,

In a method of compounding feed for animals in accordance with the present invention, the 1a-hydrorylated vitamin D compounds utilized is incorporated with the animal feed in an amount of from 5µg/kg to 40µg/kg feed on a dry weight basis. The feed mixture is then fed as a mash or as formed into desired discrete shapes for further processing and packaging. In general, these discrete shapes may be pellets, blocks or briquettes formed by known extrusion and/or compacting techniques. The particular processing technique utilized does not affect the performance of the 1α-hydroxylated vitamin D compounds in the animal feed mixture. The present invention is more specifically described by the following examples, which are meant to be illustrative only.

### Chick Efficacy Trials

### A. Procedures:

The best measure of P (or Ca) activity in animals fed a P-deficient diet is total bone ash. In the present bioassay system, young chicks (8 d of age) are fed a corn-soybean meal diet containing 0.6% Ca and 0.43% total P, but an estimated 0.10% bioavailable P. The required levels of Ca and P for chicks of this age are 1.0% Ca and 0.45% available P (i.e., nonphytate P). Calcium is kept at 0.6% instead of 1.0% in our diet because excess Ca in the presence of a severe available P deficiency causes anorexia.

Generally speaking, three or four pens of three or four chicks per pen are placed on each dietary treatment. They are fed the experimental diets free choice for 12 d in wire-screened battery pens located in a environmentally controlled animal room with constant (fluorescent) lighting. At assay termination on d 20 posthatching chicks are killed by cervical dislocation and the left tibia is quantitatively removed. Bones are stripped of adhering tissue, dried for 24 h at 100°C, weighed and then dry ashed for 24 h at 600°C (muffle furnace). The portion remaining after ashing is entirely inorganic matter. The weight of ash (mineral matter) as a percent of dry bone weight is percent ash (mineral, and mostly Ca and P) in the bone. Percent ash multiplied by dry bone weight gives total bone ash in milligrams. Tibia ash reflects the degree of ash (or bone mineralization) in the entire skeleton. Our 20-d-old crossbred chicks (New Hampshire x Columbian) fed a diet adequate in Ca and P generally have percent bone ash values of 45%.

For assessment of Zn and Mn bioavailability, bone content of Zn and Mn are the established criteria, but growth responses are also used for assessment of Zn bioavailability (Wedekind et al, 1992; Halpin and Baker, 1986). For assessment of Zn or Mn bioavailability, the tibiae are dried at 100°C for 24 h, weighed, and then dry ashed at 600°C for another 24 h. The dried ash is then wet ashed with HNO₃ and H₂O₂. Zinc and manganese are then quantified using atomic absorption spectrophotometry (Wedekind et al, 1992). In research involving Zn, Mn or Fe (hemoglobin assay) bioavailability, the chicks are fed a pretest diet (0 to d-8 posthatching) that is deficient in Zn, Mn or Fe. This depletes stores of these trace elements. The experiments are then carried out in stainless-steel chick batteries equipped with stainless-steel feeders and waterers. Deionized water is available free choice. These steps are taken to avoid Zn, Mn or Fe contamination from the environment, equipment and drinking water.

### B. Results:

The basal diet for the first experiment was designed to be severely deficient in available P (most coming from phytatebound P) but adequate to excess in vitamin D₃, and marginal in both Zn and Mn (i.e., no supplemental Zn or Mn in diet). Increases in bone ash would indicate enhanced GI absorption of P, and increases in bone Zn and Mn would indicate enhanced GI absorption of Zn and Mn (Chung and Baker, 1990; Wedekind et al, 1992; Halpin and Baker, 1986; Baker et al, 1986). As shown in Table 1, growth rate was increased (P<0.05) 17% by 0.10% P addition, 20% by 1200 U phytase addition, 15.5% by 1,25-(OH)₂D₃ addition, and 25% by the combination of phytase (1200 U) and 10.0 µg/kg 1,25-(OH)₂D₃. Bone ash, however, is the best measure of P bioavailability. Total bone ash (mg) was increased (P<0.01) 56% by 0.10% P addition (proving that P was severely deficient in the diet), 64% with 1200 U phytase, 60% by 1.25-(OH)₂D₃, and 98% by the combination of phytase and 1,25-(OH)₂D₃. Tibia Zn (µg) was increased (P<0.01) 55% by either 1200 U phytase or 10 µg/kg 1,25-(OH)₂D₃, but was increased 86% by the phytase-di-OH D₃ combination. Tibia Mn (µg) was increased (P<0.01) 63% by phytase, 85% by di-OH D₃ and 123% by the phytase-di-OH D₃ combination.

Data in Table 2 show results of a second efficacy trial. The basal diet for this trial was made adequate in Ca, and also was fortified with normal (safety factor) levels of Mn and Zn. It was thus singly deficient in available P. Bone ash was markedly depressed in chicks fed the P-deficient negative control diet. In fact, bone ash percent was about 5% lower (30.4% in Exp. 1, 25.5% in Exp. 2) in these chicks, a reflection of the high ratio of Ca to available P. Efficacy was again demonstrated for both phytase and 1,25-(OH)₂D₃. Moreover, the diet containing both phytase and 1,25-(OH)₂D₃ produced both ash values that were not far from those achieved with a P adequate diet (diet 5).

Data in Table 3 show results of a classic Zn efficacy trial. The basal diet was singly deficient in Zn (the NRC 1994 Zn requirement is 40 ppm) so that even with 10 ppm Zn addition, the diet was still Zn deficient. Marked efficacy was observed for both phytase and 1,25-(OH)₂D₃, and additivity was again evident for the combination.

Having shown conclusively that 1,25-(OH)₂D₃ is markedly efficacious in utilization of P, Zn and Mn, a trial was next conducted to test the efficacy of 1-α-OH D₃. These results are shown in Table 4, A linear (P<0.01) growth response occurred when 1-α-OH D₃ doses between 0 and 20 µg/kg were supplemented. Tibia ash likewise increased (P<0.01) markedly when 1-α-OH D₃ was added to the diet. Total tibia ash (mg) was 69% higher in chicks fed the diet with 20 µg/kg 1-α-OH D₃ than in those fed the unsupplemented basal diet. A dose of 40 µg/kg 1-α-OH D₃ was efficacious, and certainly nontoxic, but the 20 µg/kg dose maximized the response attributable to P release from phytate.

Data in Table 5 verify the synergism between the combination of microbial phytase and 1,25-(OH)₂D₃. Also, the results demonstrate that when phytase (600 vs. 1200 units) doses are compared in the presence of 10 µg/kg 1,25-(OH)₂D₃, 600 units of phytase are as effective as 1200 units in improving phytate-P utilization. This finding when compared to the data of Exp. 1 (Table 1) indicates that the phytase supplementation level required for maximum response can be cut in half if a supplemental bioactive 1-α-OH vitamin D compound is also included in the diet. In fact, only 300 units of phytase produced a marked response in the presence of 1,25-(OH)₂D₃.

Data in Table 6 show that synergism exists between 1-α-OH D₃ and phytase. Thus, 20 µg/kg 1-α-OH D₃ combined with 1200 units of phytase increased total bone ash by 107% over that observed for the basal unsupplemented corn-soybean meal diet. Supplemental 1-α-OH D₃ alone increased bone ash by 74%, and supplemental phytase alone increased bone ash by 65%.

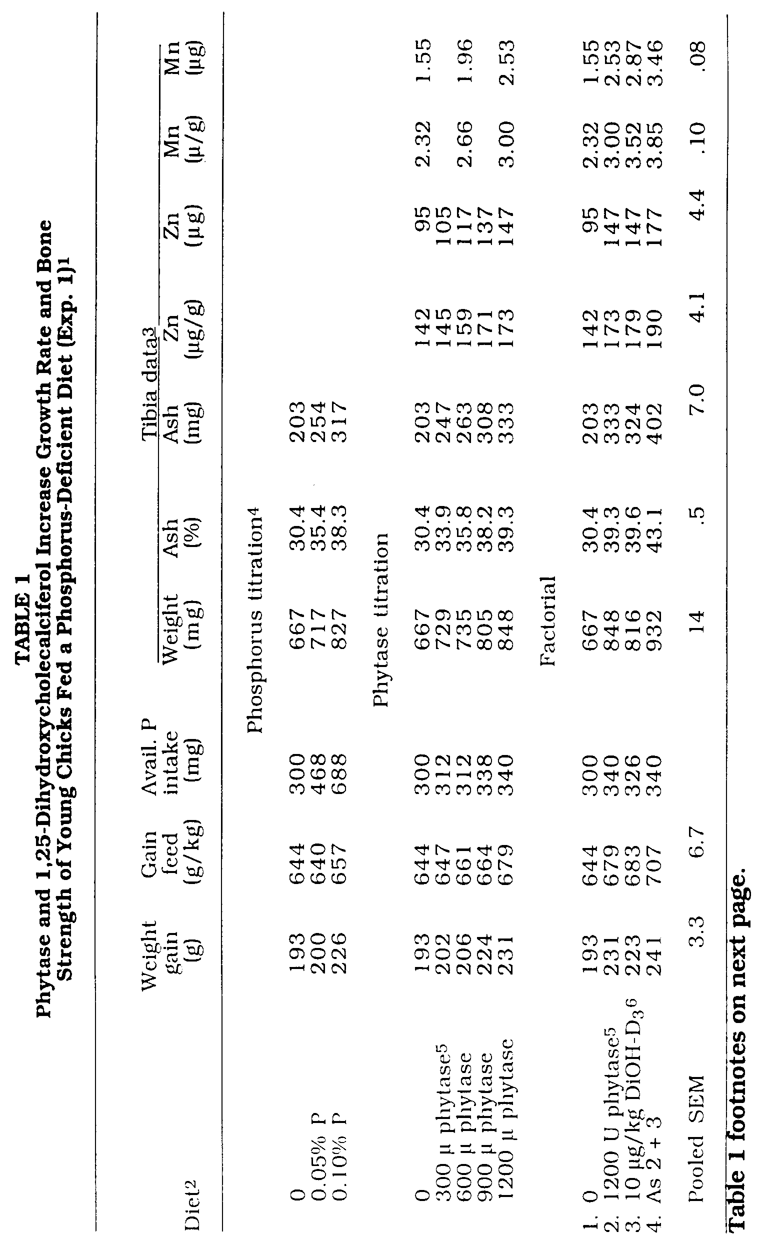

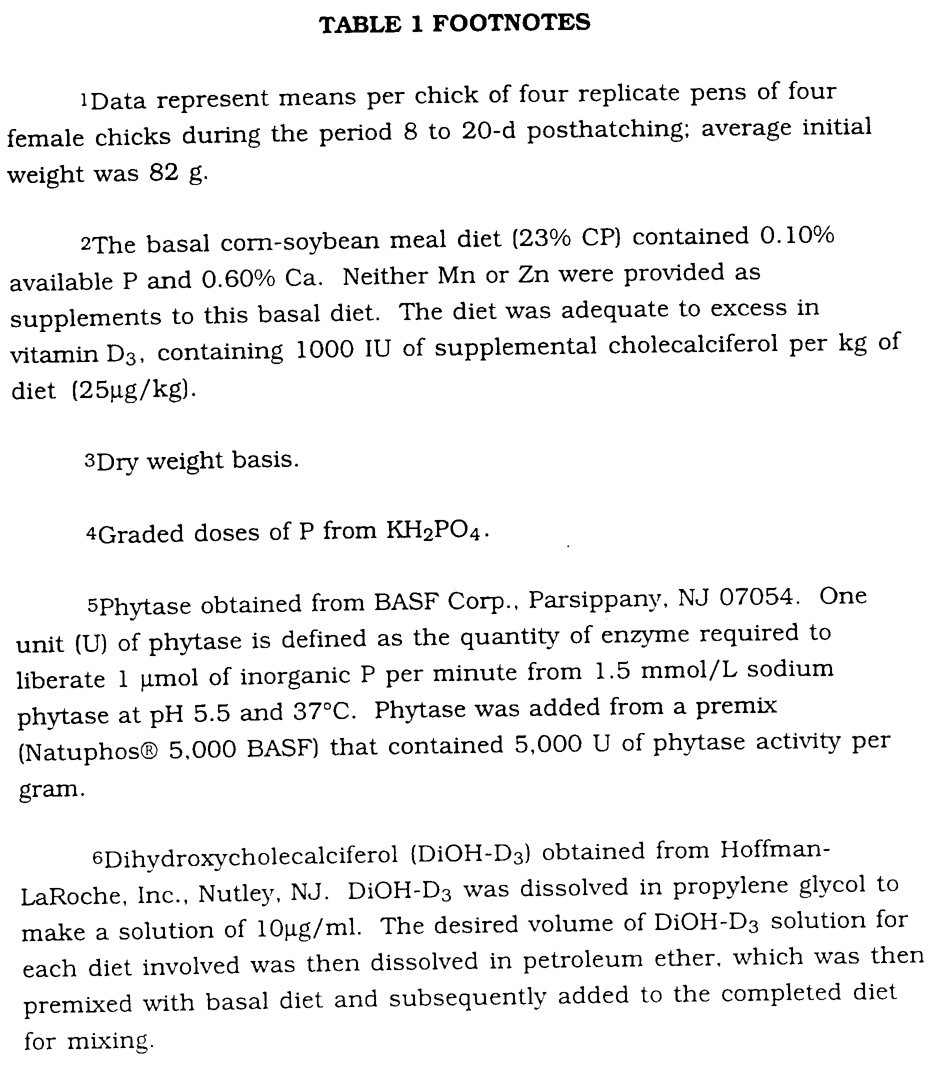

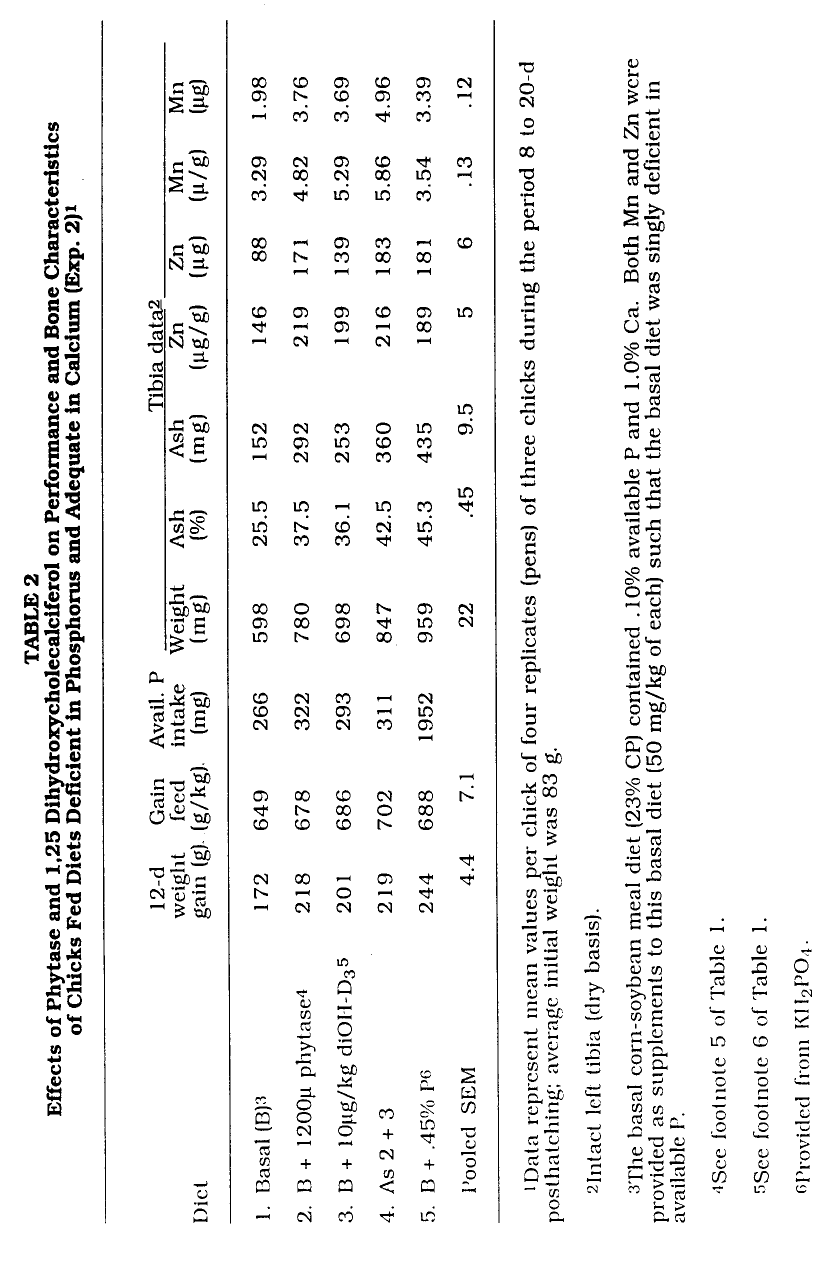

**TABLE 3**

| **Efficacy of Phytase and 1,25 Di-OH-D**_{**3**} **in Chicks Fed a Zn-Deficient Diet (Exp. 3)**^{**1**} | | | |
|---|---|---|---|
| Diet² | 12 days gain (g) | Tibia Zn (µg/g) | Tibia Zn (µg) |
| 1. Basal diet | 169 | 44.7 | 34.2 |
| 2. As 1 + 1200 U phytase | 209 | 62.2 | 54.9 |
| 3. As 1 + 10µg/kg Di-OH-D₃ | 201 | 60.3 | 53.1 |
| 4. As 2 + 3 | 241 | 88.4 | 88.7 |
| 5. As 1 + 5 ppm Zn (ZnSO₄·7H₂O) | 210 | 61.5 | 54.2 |
| 6. As 2 + 10 ppm Zn (ZnSO₄·7H₂O) | 236 | 73.7 | 71.1 |
| Pooled SEM | 8 | | 2.7 |

| | | | |
|---|---|---|---|
| ¹Data are means of four pens, each containing four male chicks weighing 84.5 g at day 8 posthatching; 12-d feeding period in stainless-steel batteries with chicks receiving deionized water, During the 8-d pretest period, chicks were fed a low Zn soybean meal diet. | | | |
| ²Soy concentrate-dextrose diet containing 13 ppm Zn. | | | |

**TABLE 4**

| **Dietary Addition of 1-α-hydroxycholecalciferol Increases Phytate-Phosphorus Utilization (Exp. 4)**^{**1**} | | | | | |
|---|---|---|---|---|---|
| Dietary Level of 1-α-OH-D₃ (µg/kg) | 12-d weight gain³ (g) | Gain feed³ (g/kg) | Tibia Data³ | | |
| | | | Weight (mg) | Ash (%) | Ash (mg) |
| 0 | 228^{b} | 645^{b} | 724^{c} | 33.0^{b} | 238^{c} |
| 10 | 255^{a} | 676^{a} | 917^{b} | 38.9^{a} | 356^{b} |
| 20 | 266^{a} | 681^{a} | 992^{a} | 40.5^{a} | 402^{a} |
| 40 | 255^{a} | 677^{a} | 878^{b} | 41.1^{a} | 361^{b} |
| Pooled SEM | 3.6 | 6.5 | 21 | .75 | 7.6 |

| | | | | | |
|---|---|---|---|---|---|
| ¹Means of three pens of four chicks during the period 8 to 20 days posthatching. | | | | | |
| ²Added to a corn-soybean meal diet (23% CP) containing adequate vitamin D-3, 0.60% Ca and 0.43% P (0.10% estimated available P). | | | | | |
| ³Means within columns with unlike superscript letters are significantly (P < 0.5) different. | | | | | |

**TABLE 5**

| **Performance and Bone Ash of Chicks Fed 1,25-Dihydroxycholecalciferol in the Absence or Presence of Three Levels of Microbial Phytase (Exp.5)**^{**1**} | | | | | |
|---|---|---|---|---|---|
| Dietary addition² | Weight gain³ (g) | Food intake (g) | Tibia data³ | | |
| | | | Weight (mg) | Ash (%) | Ash (mg) |
| 1. None | 203^{c} | 314^{c} | 672^{c} | 32.9⁴ | 238^{d} |
| 2. 10µg/kg di-OH-D₃ | 234^{b} | 338^{b} | 825^{b} | 42.2^{c} | 348^{c} |
| 3. As 2 + 300 U phytase | 244a | 349^{a,b} | 881^{a,b} | 42.5^{b,c} | 375^{b} |
| 4. As 2 + 6000 U phytase | 251^{a} | 361^{a} | 903^{a} | 43.9^{a,b} | 396^{a,b} |
| 5. As 2 + 1200 U phytase | 252^{a} | 356^{a} | 886^{a} | 44.7^{a} | 396^{a,b} |
| Pooled SEM | 3.6 | 4.6 | 20 | 0.5 | 9.0 |

| | | | | | |
|---|---|---|---|---|---|
| ¹Data are means for four pens of four female chicks that were fed the experimental diets during the period 8 to 20 d posthatching; average initial weight was 93 g. Means in columns with different superscripts letters are significantly different (P < 0.05). | | | | | |
| ²The basal diet (Table 1) contained, by analysis, 0.43% P (0.10% estimated available P), 0.63% Ca and 23% crude protein. | | | | | |
| ³Dry-weight basis. | | | | | |

**TABLE 6**

| **Evaluation of 1-α-Hydroxycholecalciferol With and Without Phytase on Phosphorus Utilization**^{**1**} | | | | | |
|---|---|---|---|---|---|
| Dietary addition | Weight gain | Food intake | Tibia data | | |
| | | | Weight | Ash | Ash |
| | g | g | mg | g/100 g | mg |
| 1. None | 195^{c} | 306^{b} | 634^{c} | 29.1^{c} | 185^{c} |
| 2. 0.10g P/100g (KH₂PO₄) | 239^{a,b} | 355^{a} | 801^{b} | 38.7^{b} | 310^{b} |
| 3. 1200 U phytase | 245^{a,b} | 356^{a} | 795^{b} | 38.5^{b} | 306^{b} |
| 4. 20 µg/kg 1-α-OH-D₃ | 235^{b} | 343^{a} | 787^{b} | 40.9^{a} | 321^{b} |
| 5. As 3 + 4 | 253^{a} | 363^{a} | 897^{a} | 42.7^{a} | 384^{a} |
| Pooled SEM | 5.5 | 6.6 | 18 | 0.7 | 11 |

| | | | | | |
|---|---|---|---|---|---|
| ¹Data are means of three pens of four female chicks that are fed the experimental diets during the period 8 to 20 d posthatching; average initial weight was 88 g. Means in columns with different superscript letters are significantly different (P < 0.05). | | | | | |
| ²The basal corn-soybean meal diet contained, by analysis, 0.43 g P/100 g (0.10 g/100 g estimated nonphytate P), 0.63 g Ca/100 g and 23.9 g CP/100g. | | | | | |
| ³Dry-weight basis. | | | | | |

## Claims

1. A method of compounding feed for fowl, comprising incorporating in a diet for fowl containing 0.5% to 1.9% of the diet an inorganic phosphorus supplement, a 1α-hydroxylated vitamin D compound so as to enhance the utilization of said inorganic phosphorus supplement and forming the resulting mixture into a discrete shape suitable for fowl.

2. The method of claim 1 wherein said discrete shape is formed by extruding said mixture.

3. The method of claim 1 wherein said discrete shape is formed by compacting said mixture.

4. The method of any one of claims 1 to 3 wherein said effective amount of the 1α-hydroxylated vitamin D compound comprises 5µg/kg to 40µg/kg of diet.

5. The method of any one of claims 1 to 4 further including the step of incorporating an effective amount of phytase with said diet.

6. The method of claim 5 wherein said effective amount of phytase comprises from 300 units to 1,200 units.

7. The method of claim 6 wherein said effective amount of phytase comprises about 600 units per kilogram of diet.

8. The method of any one of claims 1 to 7 wherein said 1α-hydroxylated vitamin D compound is characterized by the following general structure: 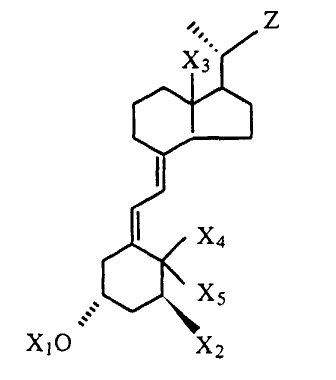 where X₁ may be hydrogen or a hydroxy-protecting group, X₂ may be hydroxy, or protected hydroxy, X₃ may be hydrogen or methyl, X₄ and X₅ each represent hydrogen or taken together X₄ and X₅ represent a methylene group, and where Z is selected from Y, -OY, -CH₂OY,-C≡CY and-CH=CHY, where the double bond may have the cis or trans stereochemical configuration, and where Y is selected from hydrogen, methyl, -CR₅O and a radical of the structure: 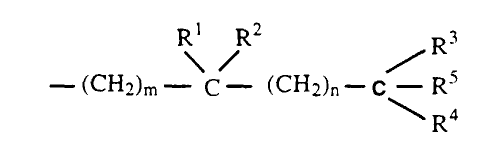 where m and n, independently, represent integers from 0 to 5, where R¹ is selected from hydrogen, hydroxy, protected-hydroxy, fluoro, trifluoromethyl, and C₁₋₅-alkyl, which may be straight chain or branched and, optionally, bear a hydroxy or protected-hydroxy substituent, and where each of R², R³ and R⁴, independently, is selected from hydrogen, fluoro, trifluoromethyl and C₁₋₅ alkyl, which may be straight-chain or branched, and optionally bear d hydroxy or protected-hydroxy substituent, and where R¹ and R², taken together, represent an oxo group, or an alkylidene group, =CR₂R₃, or the group -(CH₂)ₚ-, where p is an integer from 2 to 5, and where R³ and R⁴, taken together, represent an oxo group, or the group -(CH₂)_{q}-, where q is an integer from 2 to 5, and where R⁵ presents hydrogen, hydroxy, protected-hydroxy, or C₁₋₅ alkyl.

9. The method of claim 8 wherein the vitamin D compound is 1α-hydroxyvitamin D₃.

10. The method of claim 8 wherein the vitamin D compound is 1α,25-dihydroxyvitamin D₃.

11. A feed composition for fowl comprising: phosphorus available from an organic phosphorus source and an inorganic phosphorus supplement, said supplement supplying 0.5% to 1.9% phosphorus by weight in the diet; and
an effective amount of an 1α-hydroxylated vitamin D compound for enhancing utilization of phosphorus from said inorganic phosphorus supplement in said diet.

12. The composition of claim 11 wherein said effective amount of the 1α-hydroxylated vitamin D compound comprises 5µg/kg to 40µg/kg of diet.

13. The composition of claim 11 or 12 further including from 300 units to 1,200 units phytase in said diet.

14. The composition of claim 13 further including about 600 units phytase per kilogram of diet.

15. The composition of any one of claims 11 to 14 wherein said 1α-hydroxylated vitamin D compound is characterized by the following general structure: 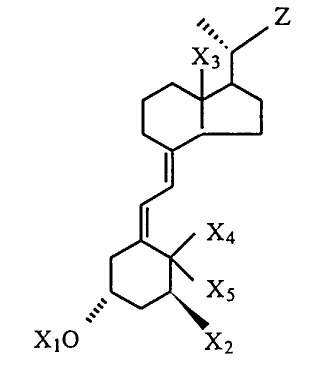 where X₁ may be hydrogen or a hydroxy-protecting group, X₂ may be hydroxy, or protected hydroxy, X₃ may be hydrogen or methyl, X₄ and X₅ each represent hydrogen or taken together X₄ and X₅ represent a methylene group, and where Z is selected from Y, -OY, -CH₂OY,-C≡CY and-CH=CHY, where the double bond may have the cis or trans stereochemical configuration, and where Y is selected from hydrogen, methyl, -CR₅O and a radical of the structure: 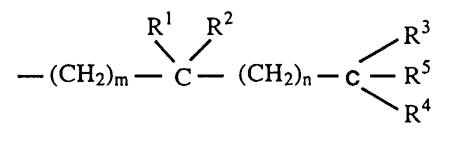 where m and n, independently, represent integers from 0 to 5, where R¹ is selected from hydrogen, hydroxy, protected-hydroxy, fluoro, trifluoromethyl, and C₁₋₅-alkyl, which may be straight chain or branched and, optionally, bear a hydroxy or protected-hydroxy substituent, and where each of R², R³ and R⁴, independently, is selected from hydrogen, fluoro, trifluoromethyl and C₁₋₅ alkyl, which may be straight-chain or branched, and optionally bear a hydroxy or protected-hydroxy substituent and where R¹ arid R², taken together, represent an oxo group, or an alkylidene group, =CR₂R₃, or the group -(CH₂)ₚ-, where p is an integer from 2 to 5, and where R³ and R⁴, taken together, represent an oxo group, or the group -(CH₂)_{q}-, where q is an integer from 2 to 5, and where R⁵ presents hydrogen, hydroxy, protected-hydroxy, or C₁₋₅ alkyl.

16. The composition of claim 15 wherein the vitamin D compound is 1α-hydroxyvitamin D₃.

17. The composition of claim 15 wherein the vitamin D compound is 1α,25-dihydroxyvitamin D₃.

18. A method of minimizing dietary requirements of phosphorus in fowl comprising the steps of:
feeding a diet containing phosphorus available from an organic phosphorus source and an inorganic phosphorus supplement, said supplement supplying 0.5% to 1.9% phosphorus by weight in the diet and
feeding with said diet an effective amount of 1α-hydroxylated vitamin D compound for enhancing utilization of phosphorus from said inorganic phosphorus supplement in said diet.

19. A method of inducing normal growth in fowl on a low phosphorus diet comprising the steps of
feeding a diet containing phosphorus to a fowl, said phosphorus available from an inorganic phosphorus supplement providing 0.5% to 1.9% by weight with the remainder being available from organic phosphorus sources;
incorporating with said diet an effective amount of a 1α-hydroxylated vitamin D compound for enhancing utilization of phosphorus from said inorganic phosphorus supplement.

20. The method of claim 18 or 19 wherein said 1α-hydroxylated vitamin D compound is fed as a top dressing on said diet.

21. The method of any one of claims 18 to 20 wherein said effective amount of the 1α-hydroxylated vitamin D compound comprises 5µg/kg to 40µg/kg of diet.

22. The method of any one of claims 18 to 21 further including the step of incorporating an effective amount of phytase with said diet.

23. The method of any one of claims 18 to 22 wherein said effective amount of phytase comprises from 300 units to 1,200 units in said diet.

24. The method of claim 23 wherein said effective amount of phytase comprises about 600 units per kilogram of diet.

25. The method of any one of claims 18 to 24 wherein said 1α-hydroxylated vitamin D compound is characterized by the following general formula: 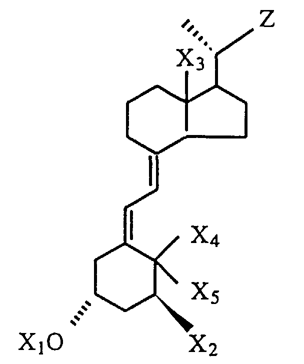 where X₁ may be hydrogen or a hydroxy-protecting group, X₂ may be hydroxy, or protected hydroxy, X₃ may be hydrogen or methyl, X₄ and X₅ each represent hydrogen or taken together X₄ and X₅ represent a methylene group, and where Z is selected from Y, -OY, -CH₂OY,-C≡CY and-CH=CHY, where the double bond may have the cis or trans stereochemical configuration, and where Y is selected from hydrogen, methyl, -CR₅O and a radical of the structure: 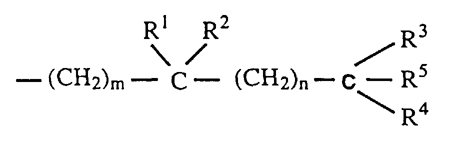 where m and n, independently, represent integers from 0 to 5, where R¹ is selected from hydrogen, hydroxy, protected-hydroxy, fluoro, trifluoromethyl, and C₁₋₅-alkyl, which may be straight chain or branched and, optionally, bear a hydroxy or protected-hydroxy substituent, and where each of R², R³ and R⁴, independently, is selected from hydrogen, fluoro, trifluoromethyl and C₁₋₅ alkyl, which may be straight-chain or branched, and optionally, bear a hydroxy or protected-hydroxy substituent, and where R¹ and R2, taken together, represent an oxo group or an alkylidene group, =CR₂R₃, or the group -(CH₂)ₚ-, where p is an integer from 2 to 5, and where R³ and R⁴, taken together represent an oxo group, or the group -(CH₂)_{q}-, where q is an integer from 2 to 5, and where R⁵ presents hydrogen, hydroxy, protected-hydroxy, or C₁₋₅ alkyl.

26. The method of claim 25 wherein the vitamin D compound is 1α-hydroxyvitamin D₃.

27. The method of claim 25 wherein the vitamin D compound is 1α,25-dihydroxyvitamin D₃.

## Patentansprüche

1. Verfahren zur Herstellung von Geflügelfutter, dadurch gekennzeichnet, daß man einem Geflügelfutter, das, bezogen auf das Futter, 0,5 % bis 1,9 % eines anorganischen Phosphorzusatzes enthält, eine 1α-hydroxylierte Vitamin D-Verbindung zusetzt, um die Verwertung des anorganischen Phosphorzusatzes zu verbessern, und die resultierende Mischung in eine für Geflügel geeignete Teilchenform bringt.

2. Verfahren nach Anspruch 1, dadurch gekennzeichnet, daß die Teilchenform durch Extrudieren der Mischung ausgebildet wird.

3. Verfahren nach Anspruch 1, dadurch gekennzeichnet, daß die Teilchenform durch Kompaktieren der Mischung ausgebildet wird.

4. Verfahren nach einem der Ansprüche 1 bis 3, dadurch gekennzeichnet, daß die wirksame Menge der 1α-hydroxylierten Vitamin D-Verbindung 5 µg/kg bis 40 µg/kg Futter beträgt.

5. Verfahren nach einem der Ansprüche 1 bis 4, dadurch gekennzeichnet, daß es außerdem die Stufe des Zugebens einer wirksamen Menge Phytase zum Futter umfaßt.

6. Verfahren nach Anspruch 5, dadurch gekennzeichnet, daß die wirksame Menge Phytase 300 Einheiten bis 1200 Einheiten beträgt.

7. Verfahren nach Anspruch 6, dadurch gekennzeichnet, daß die wirksame Menge Phytase ca. 600 Einheiten pro kg Futter beträgt.

8. Verfahren nach einem der Ansprüche 1 bis 7, dadurch gekennzeichnet, daß die 1α-hydroxylierte Vitamin D-Verbindung durch die folgende allgemeine Formel gekennzeichnet ist: 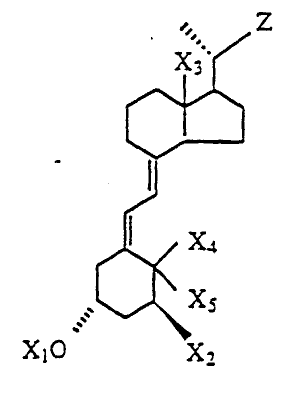 worin X₁ Wasserstoff oder eine Hydroxy-Schutzgruppe ist, X₂ Hydroxy oder geschütztes Hydroxy ist, X₃ Wasserstoff oder Methyl ist, X₄ und X₅ jeweils Wasserstoff bedeuten, oder X₄ und X₅ zusammen eine Methylengruppe bedeuten, und Z ausgewählt ist aus Y, -OY, -CH₂OY, -C≡CY und -CH=CHY, worin die Doppelbindung die stereochemische cis- oder trans-Konfiguration aufweisen kann, und worin Y ausgewählt ist aus Wasserstoff, Methyl, -CR₅O und einem Radikal der Struktur: 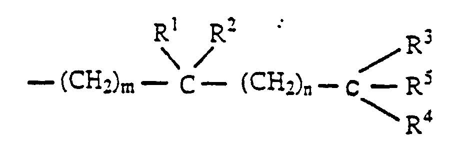 worin m und n unabhängig voneinander ganze Zahlen von 0 bis 5 bedeuten, R¹ ausgewählt ist aus Wasserstoff, Hydroxy, geschütztem Hydroxy, Fluor, Trifluormethyl und C₁₋₅-Alkyl, das geradkettig oder verzweigt sein kann, und gegebenenfalls eine Hydroxy- oder geschützte Hydroxygruppe enthalten kann, und worin jeder der Reste R², R³ und R⁴ unabhängig voneinander augewählt ist aus Wasserstoff, Fluor, Trifluormethyl und C₁₋₅-Alkyl, das geradkettig oder verzweigt sein kann, und gegebenenfalls eine Hydroxy- oder geschützte Hydroxygruppe aufweisen kann, und worin R¹ und R² zusammengenommen eine Oxogruppe, eine Alkylidengruppe, =CR₂R₃, oder die Gruppe -(CH₂)ₚ- bedeuten, worin p eine ganze Zahl von 2 bis 5 ist, und worin R³ und R⁴ zusammen eine Oxogruppe oder die Gruppe -(CH₂)_{q}- bedeuten, worin q eine ganze Zahl von 2 bis 5 ist, und worin R⁵ Wasserstoff, Hydroxy, geschütztes Hydroxy oder C₁₋₅-Alkyl bedeutet.

9. Verfahren nach Anspruch 8, dadurch gekennzeichnet, daß die Vitamin D-Verbindung 1α-Hydroxyvitamin D₃ ist.

10. Verfahren nach Anspruch 8, dadurch gekennzeichnet, daß die Vitamin D-Verbindung 1α,25-Dihydroxyvitamin D₃ ist.

11. Futterzusammensetzung für Geflügel umfassend: aus einer organischen Phosphorquelle verfügbaren Phosphor, und einen anorganischen Phosphorzusatz, wobei der Zusatz 0,5 bis 1,9 Gew.-% Phosphor im Futter bereitstellt, und eine wirksame Menge einer 1α-hydroxylierten Vitamin D-Verbindung, um die Verwertung des Phosphors aus dem anorganischen Phosphorzusatz im Futter zu verbessern.

12. Zusammensetzung nach Anspruch 11, dadurch gekennzeichnet, daß die wirksame Menge der 1α-hydroxylierten Vitamin D-Verbindung 5 µg/kg bis 40 µg/kg Futter beträgt.

13. Zusammensetzung nach Anspruch 11 oder 12, dadurch gekennzeichnet, daß sie außerdem 300 Einheiten bis 1200 Einheiten Phytase im Futter enthält.

14. Zusammensetzung nach Anspruch 13, dadurch gekennzeichnet, daß sie ca. 600 Einheiten Phytase pro kg Futter enhält.

15. Zusammensetzung nach einem der Ansprüche 11 bis 14, dadurch gekennzeichnet, daß die 1α-hydroxylierte Vitamin D-Verbindung durch die folgende allgemeine Formel charakterisiert ist: 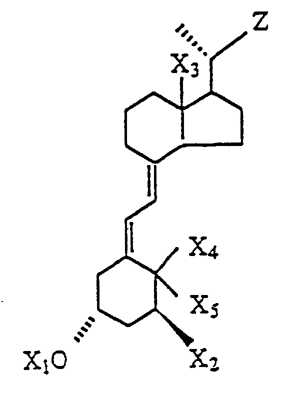 worin X₁ Wasserstoff oder eine Hydroxy-Schutzgruppe ist, X₂ Hydroxy oder geschütztes Hydroxy ist, X₃ Wasserstoff oder Methyl ist, X₄ und X₅ jeweils Wasserstoff bedeuten, oder X₄ und X₅ zusammen eine Methylengruppe bedeuten, und Z ausgewählt ist aus Y, -OY, -CH₂OY, -C≡CY und -CH=CHY, worin die Doppelbindung die stereochemische cis- oder trans-Konfiguration aufweisen kann, und worin Y ausgewählt ist aus Wasserstoff, Methyl, -CR₅O und einem Radikal der Struktur: 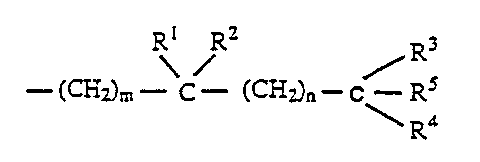 worin m und n unabhängig voneinander ganze Zahlen von 0 bis 5 bedeuten, R¹ ausgewählt ist aus Wasserstoff, Hydroxy, geschütztem Hydroxy, Fluor, Trifluormethyl und C₁₋₅-Alkyl, das geradkettig oder verzweigt sein kann, und gegebenenfalls eine Hydroxy- oder geschützte Hydroxygruppe enthalten kann, und worin jeder der Reste R², R³ und R⁴ unabhängig voneinander augewählt ist aus Wasserstoff, Fluor, Trifluormethyl und C₁₋₅-Alkyl, das geradkettig oder verzweigt sein kann, und gegebenenfalls eine Hydroxy- oder geschützte Hydroxygruppe aufweisen kann, und worin R¹ und R² zusammengenommen eine Oxogruppe, eine Alkylidengruppe, =CR₂R₃, oder die Gruppe -(CH₂)ₚ- bedeuten, worin p eine ganze Zahl von 2 bis 5 ist, und worin R³ und R⁴ zusammen eine Oxogruppe oder die Gruppe -(CH₂)_{q}- bedeuten, worin q eine ganze Zahl von 2 bis 5 ist, und worin R⁵ Wasserstoff, Hydroxy, geschütztes Hydroxy oder C₁₋₅-Alkyl bedeutet.

16. Zusammensetzung nach Anspruch 15, dadurch gekennzeichnet, daß die Vitamin D-Verbindung 1α-Hydroxyvitamin D₃ ist.

17. Zusammensetzung nach Anspruch 15, dadurch gekennzeichnet, daß die Vitamin D-Verbindung 1α,25-Dihydroxyvitamin D₃ ist.

18. Verfahren zur Minimierung des erforderlichen Phosphorbedarfes bei Geflügel umfassend die Stufen: Verfüttern eines Futters, das aus einer organischen Phosphorquelle verfügbaren Phosphor und einen anorganischen Phosphorzusatz enthält, wobei der Zusatz 0,5 bis 1,9 Gew.-% Phosphor im Futter bereitstellt, und Verfüttern einer wirksamen Menge einer 1α-hydroxylierten Vitamin D-Verbindung zusammen mit dem Futter, um die Verwertung des Phosphors aus dem anorganischen Phosphorzusatz im Futter zu verbessern.

19. Verfahren zum Herbeiführen eines normalen Wachstums bei Geflügel, dem ein Futter mit niedrigem Phosphorgehalt verabreicht wird, umfassend die Stufen:
Verfüttern eines Phosphor enthaltenden Futters an das Geflügel, wobei der Phosphor aus einem anorganischen Phosphorzusatz verfügbar ist, der 0,5 bis 1,9 Gew.-% bereitstellt, und der Rest aus organischen Phosphorquellen verfügbar ist;
Zumischen einer wirksamen Menge einer 1α-hydroxylierten Vitamin D-Verbindung zu diesem Futter, um die Verwertung des Phosphors aus dem anorganischen Phosphorzusatz zu verbessern.

20. Verfahren nach Anspruch 18 oder 19, dadurch gekennzeichnet, daß die 1α-hydroxylierte Vitamin D-Verbindung auf die Oberfläche des Futters gestreut verfüttert wird.

21. Verfahren nach einem der Ansprüche 18 bis 20, dadurch gekennzeichnet, daß die wirksame Menge der 1α-hydroxylierten Vitamin D-Verbindung 5 µg/kg bis 40 µg/kg Futter beträgt.

22. Verfahren nach einem der Ansprüche 18 bis 21, dadurch gekennzeichnet, daß es außerdem die Stufe des Zumischens einer wirksamen Menge Phytase zum Futter umfaßt.

23. Verfahren nach einem der Ansprüche 18 bis 22, dadurch gekennzeichnet, daß die wirksame Menge Phytase im Futter 300 Einheiten bis 1200 Einheiten beträgt.

24. Verfahren nach Anspruch 23, dadurch gekennzeichnet, daß die wirksame Menge Phytase ca. 600 Einheiten pro kg Futter beträgt.

25. Verfahren nach einem der Ansprüche 18 bis 24, dadurch gekennzeichnet, daß die 1α-hydroxylierte Vitamin D-Verbindung durch die folgende allgemeine Formel charakterisiert ist: 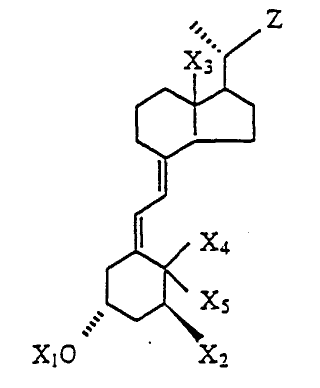 worin X₁ Wasserstoff oder eine Hydroxy-Schutzgruppe ist, X₂ Hydroxy oder geschütztes Hydroxy ist, X₃ Wasserstoff oder Methyl ist, X₄ und X₅ jeweils Wasserstoff bedeuten, oder X₄ und X₅ zusammen eine Methylengruppe bedeuten, und Z ausgewählt ist aus Y, -OY, -CH₂OY, -C≡CY und -CH=CHY, worin die Doppelbindung die stereochemische cis- oder trans-Konfiguration aufweisen kann, und worin Y ausgewählt ist aus Wasserstoff, Methyl, -CR₅O und einem Radikal der struktur: 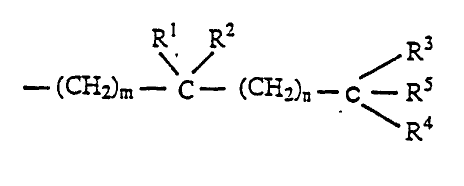 worin m und n unabhängig voneinander ganze Zahlen von 0 bis 5 bedeuten, R¹ ausgewählt ist aus Wasserstoff, Hydroxy, geschütztem Hydroxy, Fluor, Trifluormethyl und C₁₋₅-Alkyl, das geradkettig oder verzweigt sein kann, und gegebenenfalls eine Hydroxy- oder geschützte Hydroxygruppe enthalten kann, und worin jeder der Reste R², R³ und R⁴ unabhängig voneinander augewählt ist aus Wasserstoff, Fluor, Trifluormethyl und C₁₋₅-Alkyl, das geradkettig oder verzweigt sein kann, und gegebenenfalls eine Hydroxy- oder geschützte Hydroxygruppe aufweisen kann, und worin R¹ und R² zusammengenommen eine Oxogruppe, eine Alkylidengruppe, =CR₂R₃, oder die Gruppe -(CH₂)ₚ- bedeuten, worin p eine ganze Zahl von 2 bis 5 ist, und worin R³ und R⁴ zusammen eine Oxogruppe oder die Gruppe -(CH₂)_{q}- bedeuten, worin q eine ganze Zahl von 2 bis 5 ist, und worin R⁵ Wasserstoff, Hydroxy, geschütztes Hydroxy oder C₁₋₅-Alkyl bedeutet.

26. Verfahren nach Anspruch 25, dadurch gekennzeichnet, daß die Vitamin D-Verbindung 1α-Hydroxyvitamin D₃ ist.

27. Verfahren nach Anspruch 25, dadurch gekennzeichnet, daß die Vitamin D-Verbindung 1α,25-Dihydroxyvitamin D₃ ist.

## Revendications

1. Procédé de fabrication de nourriture pour volailles, qui comporte le fait d'incorporer, dans un aliment pour volailles contenant de 0,5 à 1,9 %, par rapport à l'aliment, d'un complément de phosphore inorganique, un dérivé 1α-hydroxylé de vitamine D, pour promouvoir l'utilisation dudit complément de phosphore inorganique, et le fait de mettre le mélange résultant sous forme de morceaux séparés, appropriés pour des volailles.

2. Procédé conforme à la revendication 1, dans lequel on obtient lesdits morceaux séparés en extrudant ledit mélange.

3. Procédé conforme à la revendication 1, dans lequel on obtient lesdits morceaux séparés en comprimant ledit mélange.

4. Procédé conforme à l'une des revendications 1 à 3, dans lequel ladite quantité efficace de dérivé 1α-hydroxylé de vitamine D représente de 5 à 40 µg par Kilogramme d'aliment.

5. Procédé conforme à l'une des revendications 1 à 4, qui comporte en outre une étape où l'on incorpore dans ledit aliment une quantité efficace de phytase.

6. Procédé conforme à la revendication 5, dans lequel ladite quantité efficace de phytase vaut de 300 à 1200 unités.

7. Procédé conforme à la revendication 6, dans lequel ladite quantité efficace de phytase vaut à peu près 600 unités par Kilogramme d'aliment.

8. Procédé conforme à l'une des revendications 1 à 7, dans lequel ledit dérivé 1α-hydroxylé de vitamine D est caractérisé par la structure générale suivante : 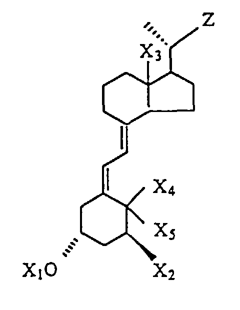 dans laquelle X₁ peut représenter un atome d'hydrogène ou un groupe hydroxy-protecteur, X₂ peut représenter un groupe hydroxy ou un groupe hydroxy protégé, X₃ peut représenter un atome d'hydrogène ou un groupe méthyle, X₄ et X₅ représentent chacun un atome d'hydrogène ou bien, considérés conjointement, un groupe méthylène, et Z est choisi parmi -Y, -OY, -CH₂OY, -C≡CY et -CH=CHY où la double liaison peut présenter la configuration stéréochimique cis ou trans et Y représente un atome d'hydrogène, un groupe méthyle, un groupe de formule -CR₅O ou un groupe de structure 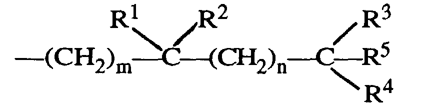 dans laquelle m et n représentent indépendamment des nombres entiers valant de 0 à 5, R¹ est choisi parmi un atome d'hydrogène ou de fluor et un groupe hydroxy, hydroxy protégé, trifluorométhyle ou alkyle en C₁₋₅, qui peut être linéaire ou ramifié et porter éventuellement un sub-stituant hydroxy ou hydroxy protégé, et chacun des symboles R², R³ et R⁴ est choisi parmi un atome d'hydrogène ou de fluor et un groupe trifluorométhyle ou alkyle en C₁₋₅, qui peut être linéaire ou ramifié et porter éventuellement un substituant hydroxy ou hydroxy protégé, R¹ et R², considérés conjointement, pouvant aussi représenter un substituant oxo, un groupe alkylidène =CR₂R₃ ou un groupe -(CH₂)ₚ- où p représente un nombre entier valant de 2 à 5, et R³ et R⁴, considérés conjointement, pouvant aussi représenter un substituant oxo ou un groupe -(CH₂)_{q}- où q représente un nombre entier valant de 2 à 5, et R⁵ représente un atome d'hydrogène ou un groupe hydroxy, hydroxy protégé ou alkyle en C₁₋₅.

9. Procédé conforme à la revendication 8, dans lequel le dérivé de vitamine D est la 1α-hydroxyvitamine D₃.

10. Procédé conforme à la revendication 8, dans lequel le dérivé de vitamine D est la 1α,25-dihydroxyvitamine D₃.

11. Composition de nourriture pour volailles, qui contient : - du phosphore apporté par une source de phosphore organique et un complément phosphoré inorganique, ledit complément apportant de 0,5 à 1,9 % en poids de phosphore dans l'aliment ; et - un dérivé 1α-hydroxylé de vitamine D, en une quantité suffisante pour promouvoir efficacement l'utilisation du phosphore tiré dudit complément phosphoré inorganique incorporé dans ledit aliment.

12. Composition conforme à la revendication 11, dans laquelle ladite quantité efficace de dérivé 1α-hydroxylé de vitamine D représente de 5 à 40 µg par kilogramme d'aliment.

13. Composition conforme à la revendication 11 ou 12, qui contient en outre de 300 à 1200 unités de phytase incorporée dans ledit aliment.

14. Composition conforme à la revendication 13, qui contient en outre à peu près 600 unités de phytase par kilogramme d'aliment.

15. Composition conforme à l'une des revendications 11 à 14, dans laquelle ledit dérivé 1α-hydroxylé de vitamine D est caractérisé par la structure générale suivante : 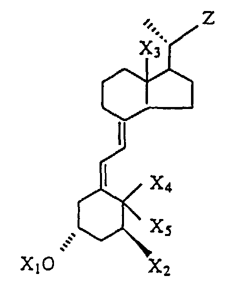 dans laquelle X₁ peut représenter un atome d'hydrogène ou un groupe hydroxy-protecteur, X₂ peut représenter un groupe hydroxy ou un groupe hydroxy protégé, X₃ peut représenter un atome d'hydrogène ou un groupe méthyle, X₄ et X₅ représentent chacun un atome d'hydrogène ou bien, considérés conjointement, un groupe méthylène, et Z est choisi parmi -Y, -OY, -CH₂OY, -C≡CY et -CH=CHY où la double liaison peut présenter la configuration stéréochimique cis ou trans et Y représente un atome d'hydrogène, un groupe méthyle, un groupe de formule -CR₅O ou un groupe de structure 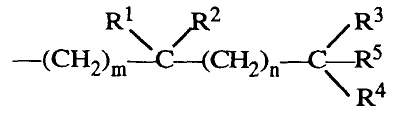 dans laquelle m et n représentent indépendamment des nombres entiers valant de 0 à 5, R¹ est choisi parmi un atome d'hydrogène ou de fluor et un groupe hydroxy, hydroxy protégé, trifluorométhyle ou alkyle en C₁₋₅, qui peut être linéaire ou ramifié et porter éventuellement un substituant hydroxy ou hydroxy protégé, et chacun des symboles R², R³ et R⁴ est choisi parmi un atome d'hydrogène ou de fluor et un groupe trifluorométhyle ou alkyle en C₁₋₅, qui peut être linéaire ou ramifié et porter éventuellement un substituant hydroxy ou hydroxy protégé, R¹ et R², considérés conjointement, pouvant aussi représenter un substituant oxo, un groupe alkylidène =CR₂R₃ ou un groupe -(CH₂)ₚ- où p représente un nombre entier valant de 2 à 5, et R³ et R⁴, considérés conjointement, pouvant aussi représenter un substituant oxo ou un groupe -(CH₂)_{q}- où q représente un nombre entier valant de 2 à 5, et R⁵ représente un atome d'hydrogène ou un groupe hydroxy, hydroxy protégé ou alkyle en C₁₋₅.

16. Composition conforme à la revendication 15, dans laquelle le dérivé de vitamine D est la 1α-hydroxyvitamine D₃.

17. Composition conforme à la revendication 15, dans laquelle le dérivé de vitamine D est la 1α,25-dihydroxyvitamine D₃.

18. Procédé permettant de réduire les besoins alimentaires en phosphore chez des volailles, qui comporte les étapes suivantes :
- donner comme nourriture un aliment qui contient du phosphore apporté par une source de phosphore organique et un complément phosphoré inorganique, ledit complément apportant de 0,5 à 1,9 % en poids de phosphore dans l'aliment ; et
- donner comme nourriture, avec ledit aliment, un dérivé 1α-hydroxylé de vitamine D, en une quantité suffisante pour promouvoir efficacement l'utilisation du phosphore tiré dudit complément phosphoré inorganique incorporé dans ledit aliment.

19. Procédé permettant de maintenir une croissance normale chez des volailles dont l'alimentation est pauvre en phosphore, lequel procédé comporte les étapes suivantes :
- donner comme nourriture à des volailles un aliment qui contient du phosphore, ce phosphore étant apporté, à raison de 0,5 à 1,9 % en poids, par un complément phosphoré inorganique, et le reste étant apporté par des sources de phosphore organique ; et
- incorporer dans ledit aliment un dérivé 1α-hydroxylé de vitamine D, en une quantité suffisante pour promouvoir efficacement l'utilisation du phosphore tiré dudit complément phosphoré inorganique.

20. Procédé conforme à la revendication 18 ou 19, dans lequel le dérivé 1α-hydroxylé de vitamine D est donné sous la forme d'un assaisonnement ajouté sur ledit aliment

21. Procédé conforme à l'une des revendications 18 à 20, dans lequel ladite quantité efficace de dérivé 1α-hydroxylé de vitamine D représente de 5 à 40 µg par kilogramme d'aliment.

22. Procédé conforme à l'une des revendications 18 à 21, qui comporte en outre une étape où l'on incorpore dans ledit aliment une quantité efficace de phytase.

23. Procédé conforme à l'une des revendications 18 à 22, dans lequel ladite quantité efficace de phytase vaut de 300 à 1200 unités.

24. Procédé conforme à la revendication 23, dans lequel ladite quantité efficace de phytase vaut à peu près 600 unités par kilogramme d'aliment.

25. Procédé conforme à l'une des revendications 18 à 24, dans lequel ledit dérivé 1α-hydroxylé de vitamine D est caractérisé par la structure générale suivante : 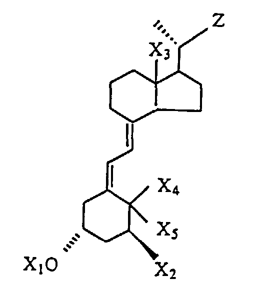 dans laquelle X₁ peut représenter un atome d'hydrogène ou un groupe hydroxy-protecteur, X₂ peut représenter un groupe hydroxy ou un groupe hydroxy protégé, X₃ peut représenter un atome d'hydrogène ou un groupe méthyle, X₄ et X₅ représentent chacun un atome d'hydrogène ou bien, considérés conjointement, un groupe méthylène, et Z est choisi parmi -Y, -OY, -CH₂OY, -C≡CY et -CH=CHY où la double liaison peut présenter la configuration stéréochimique cis ou trans et Y représente un atome d'hydrogène, un groupe méthyle, un groupe de formule -CR₅O ou un groupe de structure 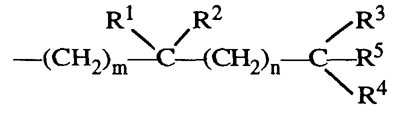 dans laquelle m et n représentent indépendamment des nombres entiers valant de 0 à 5, R¹ est choisi parmi un atome d'hydrogène ou de fluor et un groupe hydroxy, hydroxy protégé, trifluorométhyle ou alkyle en C₁₋₅, qui peut être linéaire ou ramifié et porter éventuellement un substituant hydroxy ou hydroxy protégé, et chacun des symboles R², R³ et R⁴ est choisi parmi un atome d'hydrogène ou de fluor et un groupe trifluorométhyle ou alkyle en C₁₋₅, qui peut être linéaire ou ramifié et porter éventuellement un substituant hydroxy ou hydroxy protégé, R¹ et R², considérés conjointement, pouvant aussi représenter un substituant oxo, un groupe alkylidène =CR₂R₃ ou un groupe -(CH₂)ₚ- où p représente un nombre entier valant de 2 à 5, et R³ et R⁴, considérés conjointement, pouvant aussi représenter un substituant oxo ou un groupe -(CH₂)_{q}- où q représente un nombre entier valant de 2 à 5, et R⁵ représente un atome d'hydrogène ou un groupe hydroxy, hydroxy protégé ou alkyle en C₁₋₅.

26. Procédé conforme à la revendication 25, dans lequel le dérivé de vitamine D est la 1α-hydroxyvitamine D₃.

27. Procédé conforme à la revendication 25, dans lequel le dérivé de vitamine D est la 1α,25-dihydroxyvitamine D₃.
